Europäisches Patentamt

European Patent Office

Office européen des brevets

(19)

(11) Publication number: 0 247 796
A1

(12) **EUROPEAN PATENT APPLICATION**

(21) Application number: 87304526.4

(22) Date of filing: 21.05.87

(51) Int. Cl.⁴: **G01N 33/546** , G01N 33/543 , //G01N33/553,G01N27/46,G01-N33/535,G01N33/533

The title of the invention has been amended (Guidelines for Examination in the EPO, A-III, 7.3).

(30) Priority: 22.05.86 GB 8612458
29.08.86 GB 8620894

(43) Date of publication of application:
02.12.87 Bulletin 87/49

(84) Designated Contracting States:
AT BE CH DE ES FR GB GR IT LI NL SE

(71) Applicant: **UNILEVER PLC**
**Unilever House Blackfriars P.O. Box 68**
**London EC4P 4BQ(GB)**

(84) **GB**

(71) Applicant: **UNILEVER NV**
**Burgemeester s'Jacobplein 1 P.O. Box 760**
**NL-3000 DK Rotterdam(NL)**

(84) **BE CH DE ES FR GR IT LI NL SE AT**

(72) Inventor: **Davis, Paul James**
**The Hawthorns Pavenham Road**
**Felmersham Bedfordshire MK43 7EX(GB)**
Inventor: **Drake, Rosemary Ann Lucy**
**1 Warren Cottages Church End Barley**
**Royston Hertfordshire SG8 8JP(GB)**
Inventor: **Hornby, William Edward**
**1 Ray Mill Road West Maidenhead**
**Berkshire SL6 8FA(GB)**

(74) Representative: **Butler, David John et al**
**Unilever PLC Patents Division P.O. Box 68**
**Unilever House**
**London EC4P 4BQ(GB)**

(54) **Solid phase immunoassay method.**

(57) An immunoassay for determining qualitatively or quantitatively the presence of an analyte in a sample by means of specific binding, wherein the sample is contacted with a moveable solid phase carrier material, e.g. magnetic particles (620) on which is immobilised a first binding reagent having specificity for the analyte and with a labelled reagent which can participate in either a "sandwich" or a "competition" reaction with the first reagent in the presence of the analyte and following an incubation period sufficient to allow the reaction to take place, the carrier material is moved within the assay medium (618) to a location adjacent a signal sensing means (602), the label generating a signal, such as chemiluminescent light, continuously in the assay medium and the magnitude of the signal generated in the vicinity of the sensing means being used as a measure of the extent to which the binding reaction has occurred. Preferably the assay medium incorporates a masking or quenching agent which suppresses signal generated in regions of the assay medium remote from the signal sensing means.

## ASSAYS

The present invention relates to assays utilising specific binding, such as immunoassays.

Solid phase, e.g. particle-based, immunoassay systems are already known. These involve the qualitative and/or quantitative determination of an immunogenic species in a test sample by means of an immunological binding reaction between the immunogenic species (analyte) and one or more specific binding partners therefor, at least one of which binding partners is linked to an insoluble carrier material in contact with a liquid medium comprising the test sample. The fact that the resulting immunological complex is linked to the carrier material can be used to advantage during the subsequent determination of the extent to which the binding reaction has taken place. The determination is usually achieved by the use of a labelled specific binding partner, and the binding reaction can be either a "competition" reaction or a "sandwich" reaction, both of which are now widely used in this art.

In a typical "competition" reaction, the assay medium, which is generally aqueous, is contacted with the solid phase carrier on which is immobilised a specific binding reagent (e.g. monclonal antibody) having specificity for the analyte to be determined, and the assay medium contains a known quantity of the analyte (or an analogue thereof possessing the identical epitopes) bearing a label and a known quantity of a sample suspected of containing the analyte. In the absence of analyte in the sample, the labelled analyte or analogue binds with the specific binding reagent immobilised on the solid phase carrier and the extent of this binding is determined from standard experiments. When the sample contains the analyte, this competes with the labelled analyte or analogue for the specific binding reagent and hence the extent to which the specific binding reagent becomes coupled to the labelled component is reduced. By comparing this with the standard situation, (i.e. the result obtainable in the absence of the analyte) a measure of the analyte concentration in the sample can be derived.

In a typical "sandwich" reaction, a first binding reagent having specificity for the analyte is immobilised on the solid phase carrier, and the assay medium, which is again generally aqueous, also contains a second binding reagent having specificity the analyte and which bears a label. In the absence of analyte, no coupling can occur between the immobilised reagent and the labelled reagent. When the analyte is present it acts as a bridge between the immobilised and labelled specific binding reagents and results in indirect coupling of the labelled reagent to the solid phase. The extent of this coupling provides a measure of the analyte concentration in the sample. The specificities of the immobilised reagent and the labelled reagent for the analyte can be different, but, if the analyte molecule possesses more than one identical epitopes, it is possible to use the same specific binding reagent both in the immobilised form and in the free-labelled form.

Several types of particle-based immunoassay systems are already known. Some of these involve complicated detection systems for the complex that results from the specific binding reaction, and/or the complete physical separation of the particulate carrier material carrying the complex from the test medium, including removal of all unbound material, prior to determination of the species under test. In one embodiment, the present invention seeks to provide a non-separation particle-based immunoassay systems in which the determination of the species under test can be made rapidly and simply, and especially immunoassays wherein only one step is required.

Immunoassays involving the localisation of particulate solid phase carriers within an assay medium are described in various documents. EP 0169434 describes a fluorometric immunological assay method in which an antigen is attached to magnetic particles which are, for the time of measurement, pulled against the wall of a measurement vessel by means of a magnetic field. To obtain an analytical result in such a system, it is, of course, necessary to stimulate the fluorescence by the external application of energy such UV light.

EP 0149565 describe immunoassays performed using a labelled reagent and another reagent bound to magnetically attractable particles, which are suspendable but insoluble in a liquid assay medium. After the labelled reagent has become partitioned between the liquid phase and the particles, in proportions which depend on the concentration of an analyte in a sample, the liquid phase is removed. Then the particles are resuspended in another liquid medium and the concentration of label observed. The method is said to be particularly suitable for fluorescent and chemiluminescent label systems, and can conveniently be performed in microtiter plates in which the wells are optically screened from one another, the observation being made from above or below the wells. EP 0149565 specifically states that it is difficult to make useful measurements of any optical signal generated from the pellet which results from bringing down a particulate reagent out of suspension.

The philosophy behind the invention described in EP 0149565 is completely opposite to that on which the present invention is based, because we have found that not merely is it possible but, indeed, it is very advantageous to make the relevant observations from the localised solid phase rather than from the dispersed solid phase.

The invention provides a carrier-based assay method which utilises a specific binding partner which is labelled such that the label continuously generates a signal in the test medium, and in which method, following the binding reaction, the carrier material is moved to an environment within the assay medium wherein any enhanced local signal flux can be detected.

More specifically, the invention provides an assay method for determining qualitatively and/or quantitatively the presence of an analyte in a sample by means of specific binding, wherein the sample is contacted with a moveable solid phase carrier material on which is immobilised a first binding reagent having specificity for the analyte and with a labelled specific binding reagent which can participate in either a "sandwich" or a "competition" reaction with the first reagent in the presence of the analyte, and in which method, following an incubation period sufficient to allow the reaction to take place, the moveable solid phase carrier material is moved within the assay medium to a location adjacent a signal sensing means, the label generating a signal continuously in the assay medium and the magnitude of the signal generated in the vicinity of the sensing means being used as a measure of the extent to which the binding reaction has occurred.

The invention will be described in detail principally in the context of particle-based immunoassays, but from the following description it will become apparent that other practical applications can be effected without departing from the broad spirit of the invention.

The invention provides, as one embodiment, a particle-based immunoassay method which utilises a specific binding reagent which is labelled such that the label continuously generates a signal in the assay medium, which signal is suppressed while the labelled binding reagent is uniformly dispersed in the assay medium, and in which method, following the binding reaction, the particulate carrier material is localised in an environment having insufficient signal-suppressing, ability to overcome the locally-generated signal, and hence a detectable signal can be generated the nature and/or magnitude of which is dependent on the extent of binding that has taken place between the analyte and the specific binding reagent carried by the particles.

In a more preferred embodiment, the invention provides a particle-based immunoassay method which utilises a specific binding partner which is labelled such that the label continuously generates a signal in the test medium, which signal is masked or quenched or otherwise suppressed while the labelled binding partner is uniformly dispersed within the test medium, and in which method, following the binding reaction, a local concentration of the particulate carrier material is established in the test medium such that the local concentration of complexed labelled binding partner carried by the particles provides a local signal flux that is sufficient to overcome the local signal-suppressing ability of the test medium and hence to provide a detectable signal the nature and/or magnitude of which is dependent on the extent of binding that has taken place between the immunogenic species under test and the specific binding partner carried by the particles.

In the context of the present invention, the term "signal" is used to denote any phenomenon that can be observed or measured, qualitatively or quantitatively, and the term "detectable signal" is used to denote any significant change in the signal that can be observed or measured. For example, the signal can be the concentration of a chemical entity, and the detectable signal can be an increase or decrease in concentration from a relatively uniform "background" signal (which might be zero). Another example of a detectable signal would be a change in the rate of accumulation of a chemical entity. A further example of a detectable signal would be change in the intensity of electromagnetic radiation, such as visible light, emitted by a chemical species such as a chemiluminescent agent.

An immunoassay method of the invention is therefore characterised by the features:

a. the label generates a signal continuously while in the test medium;

b. while the label is dispersed in the test medium, irrespective of whether the label is free or bound in a complex linked to the carrier material, the signal is masked or quenched or otherwise suppressed;

c. complexing of the labelled binding partner with the other binding partner does not of itself significantly alter the signal generated by the label; and

d. a detectable signal is produced only when the carrier material, to which is linked the complexed label, is localised in a comparatively small zone within the test medium.

The solid phase, e.g. the particulate carrier, initially has access to the bulk of the test medium, and hence can be under the influence of any signal-suppressing activity that the bulk medium possesses. Subsequently, the solid phase is placed in a situation wherein it has relative freedom from the bulk sample and hence is less influenced by the signal-suppressing effect. The movement of the solid phase is the only action that needs to be performed in order to give a readable result. Constant signal production by the label itself obviates the need to add further reagents or to provide further stimuli after particle localisation has taken place and provides maximum sensitivity of detection with the minimum complexity or number of operations. The simple act of physically localising the carrier particles is all that is required in order for a detectable signal to be produced.

The suppression of the signal generated by the labelled binding partner when dispersed in the test medium is a most important feature of the invention. The mechanism by which the signal is suppressed will depend on the nature of the signal generating system itself. The following mechanisms, which are given by way of example only, illustrate the range of possibilities that exist. The signal suppressing effect in the bulk sample must be balanced such that a local concentration of the label produces a detectable signal. In any given system, the level of suppression has to be determined by experiment and experience. However, this is not difficult to achieve. A dispersed mixture of the carrier material, bound signal generator and free signal generator, representing the concentration that would be expected in the non-localised sample is, "titrated" with the appropriate signal suppressing means (e.g. a consumer reagent) until the level of signal suppression is just sufficient to ensure that there is no net production of signal in the absence of localisation of the bound signal generator.

The label can be a chemical agent that reacts with a substrate present in the medium to produce a chemical product. If the test medium contains in appropriate concentration a consumer reagent that destroys all or most of the chemical product as soon as it is formed, a uniform background signal (e.g. the absolute concentration or rate of change of concentration of the chemical product) will be maintained as long as the label remains dispersed throughout the bulk of the test medium. However, following a binding reaction with a specific binding partner linked to the carrier particles, localisation of the particles within a zone in the test medium can cause the chemical product to accumulate at a rate which significantly exceeds the rate at which the consumer reagent can destroy the chemical product in that zone. The increasing quantity of chemical product in the immediate vicinity of the localised particles will constitute a detectable signal.

While the particulate carrier material is localised within a zone in the test medium, the consumer reagent must remain uniformly dispersed throughout the bulk of the test medium.

Such a chemical suppression can be achieved easily using, for example, glucose oxidase enzyme as the label, glucose as the substrate in the test medium, and catalase enzyme in the test medium as the consumer reagent. The concentration of catalase in the test medium can be chosen such that under normal circumstances, while the glucose oxidase is uniformly dispersed in the test medium, all or most of the hydrogen peroxide produced by reaction between the glucose oxidase and the glucose is immediately destroyed by the catalase. The background level of hydrogen peroxide will remain constant or will alter only slowly and uniformly. However, when the particulate carrier bearing a complex including the glucose oxidase-labelled specific binding partner is concentrated in a comparatively small zone in the test medium, the amount of hydrogen peroxide accumulated in that locality will be sufficient to overcome the action of the catalase, and can be measured as an increased rate of accumulation of the peroxide. The hydrogen peroxide can be detected, for example, by using an appropriate electrode, the localisation of the particulate carrier being effected in the immediate vicinity of the electrode. Interfering chemicals, which can be present in the sample, such as ascorbic acid or paracetamol, and which can give rise to spurious electrochemical signals like that caused by peroxide, can be consumed by means of an electrode in the cell. This may avoid the need for selective membranes protecting the working electrode.

Alternatively the chemical product "signal" can be detected via a further chemical reaction with another reagent which is localised on or in a surface. For example, the particulate carrier material to which the complex is linked can be localised adjacent a surface (such as a slab, sheet, film or membrane, or a second particulate carrier material) containing or carrying a reagent which interacts with the "signal" product and which results in, for example, the formation of a colour change in or on the surface or the emission of light from the surface. Taking as an example the hydrogen peroxide-generating system just described, the detector surface could contain or carry a peroxide-utilising enzyme, such as horseradish peroxidase (HRP), leading to the formation of a coloured product by oxidation of a substrate in or on the detector surface.

Another alternative can be the use of a label that alters the pH of the test medium, such as urease or penicillinase, with an appropriate substrate (such as urea) in the test medium. In the dispersed situation, the effect of the label can be masked or quenched by incorporating a pH buffer in the test medium. Following the binding reaction and localisation of the particles carrying the complex, the local concentration of label will cause a pH change that overcomes the buffering capacity of the test medium in the immediate vicinity of the localised carrier particles. This pH change can be detected, for example, using a pH electrode or a pH-responsive indicator paper.

In a further alternative embodiment, the sensing means comprises a gas detector located outside the vessel in which the assay is conducted. The sensor can, for example, be a chemical field effect transistor (Chemfet), sensing ammonia. The sensor can be located adjacent a gas-permeable membrane in the vessel wall, against which the carrier material is localised. The label can be urease enzyme, converting substrate urea to ammonia, and the test medium can contain l-glutamate dehydrogenase as a consumer reagent.

A system that is completely different in detail but analogous in principle, can be made using a label which is chemiluminescent or which participates in a chemiluminescent reaction with other components of the test medium. If the test medium is opaque or at least insufficiently translucent for the light to be observable when the labelled specific binding partner is evenly dispersed in the test medium, an optical signal will not be detectable. Alternatively, the test medium can contain a chemical species that interacts with a component of the light-generating system, and quenches or inhibits the production of an optical signal. Subsequent localisation of the particulate carrier to which is linked a complex involving the labelled specific binding partner can give rise to a localised optical signal which is of sufficient intensity to be detectable. The localisation can be effected, for example, adjacent a "window" in the vessel holding the test medium, or adjacent an optical sensor. The placing of a (second) optical sensor in the body of the test fluid to measure the background light intensity would be useful in providing means for calibration or normalisation.

Such a system can be provided using, for example, horseradish peroxidase as the label, with luminol and a peroxide (such as $H_2O_2$) in the medium. The medium can contain a dye, sufficiently chemically inert not to be affected by the peroxide reaction, which gives the medium an optical density at an appropriate wavelength that is sufficiently high (preferably O.D. of at least about 2) to render the light generated by the dispersed label insignificant in comparison with that observable from a localised concentration of the label. The use of dyes (attenuators) which absorb light at wavelengths including that of the emmitted luminescence in immunoassays involving luminescent reactions as the labelling system, is described in EP 0165072. Alternatively, the test medium can contain a chemical reagent that interferes with the generation of the luminescence.

A further way in which the chemiluminescent signal can be reduced or eliminated in the bulk test sample is to include a quencher reagent in the sample. For example, if horseradish peroxide enzyme is used as the label, reacting with peroxide and luminol in solution to produce visible light, the reaction can be inhibited by incorporating a competing reagent, such as gallic acid or thiodiglycollic acid, in the sample.

It can be advantageous to incorporate a fluorescer system that can absorb the chemiluminescent light and emit at a different wavelength. Such a system can help to screen out the effect of light generated in the bulk sample, especially if the emitted light is viewed through an appropriate filter. For example, blue light from luminol can be absorbed by a coumarin, which will emit yellow/green light. The fluorescer can be located on or near the signal sensing means. Alternatively, the fluorescer can be used as a marking agent dispersed in the bulk sample, so that only a localised generation of chemiluminescence is observable.

Although luminol (5-amino-2,3-dihydro-1,4-phthalazinedione) is a particularly preferred chemiluminescent agent for use in the context of the invention, a range of alternative compounds and reactions can be used. For example, other derivatives of 2,3-dihydro-1,4-phthalazinedione, such as the 6-amino derivative (isoluminol) can be used. Other examples are luciferin and acridinium esters. Such systems are generally described, for example, in GB 1552607, GB 2112779A and GB 2095830B. In general, chemiluminescent reactions tend to be short-lived, which may lead to time constraints on the assay procedure. It is therefore useful to enhance the effective denation of the light emission, e.g. by the addition of reagents that prolong or delay the emission. The enhancement of chemiluminescent reactions, using compounds such as substituted 6-hydroxybenzothiazoles, p-iodophenol, 4-phenylphenol and/or 2-chloro-4-phenylphenol and aromatic amines, is described in EP 87959, EP 116454 and GB 2162946A. The use of p-iodophenol is particularly preferred.

An important embodiment of the invention is a particle-based assay method which utilises a specific binding partner which is labelled with a reagent that participates in a chemiluminescent reaction, the other essential reagent(s) for the chemiluminescent reaction being present in abundance in the test medium, such that the chemiluminescent reaction takes place continuously in the test medium, the light so produced being masked or quenched while the labelled binding partner is uniformly dispersed within the test medium, and in which method following the binding reaction a local concentration of the particulate carrier material is established in the test medium adjacent a detection means such that the local concentration of complexed labelled binding partner carried by the particles provides a local emission of light which is sufficient to overcome the local masking or quenching ability of the test medium and hence to provide a detectable signal, the nature and or magnitude of which is dependent on the extent of binding that has taken place between the species under test and the specific binding partner carried by the particles.

A further embodiment of the invention is a particle-based assay method which utilises:

(a) a first population of localisable particles (e.g. "magnetic" particles) bearing a specific binding partner for a chemical species under test;

(b) a non-particle-borne specific binding partner labelled such that the specific binding partner produces continuously within the test medium a precursor for a chemiluminescent reaction;

(c) a consumer reagent dispersed in the test medium which competes effectively for the precursor and thereby inhibits the production of chemiluminescence while the labelled specific binding partner remains dispersed in the test medium; and

. (d) a second population of localisable particles bearing or containing a reagent which can react with the precursor to generate chemiluminescence, and in which method co-localisation of the two particle populations adjacent a light detection means enables observation of chemiluminescence produced in an amount dependent on the extent to which the specific binding partner carried by the first population of particles has bound to the species under test.

In the foregoing embodiment, the label can be, for example, glucose oxidase reacting with glucose and oxygen in the test medium to produce hydrogen peroxide as the precursor, the consumer can be catalase enzyme, and the bound reagent can be horseradish peroxidase interacting with luminol in the text medium. If desired, the bound reagent can be carried on the surface or entrapped within the particles of the second population.

Sequential addition of the components in the test medium can be advantageous, e.g. to permit adequate time ("incubation") for the immunological binding reaction(s) to take place prior to the addition of a substrate required for the generation of a signal by the label. It will thus be appreciated that the continuous generation of signal by the label will only take place when the necessary substrates have been added. This addition will always be made before the solid phase is relocated to the detection zone or environment. The continuity of the signal generation must last at least for the duration of the time taken to localise the solid phase and to detect the signal produced therefrom.

The incubation period needed for the specific binding reaction(s) to take place will depend on many factors, such as the nature of the reagents and the analyte, their relative and absolute concentrations, and on physical factors such as temperature. These parameters are familiar to thos skilled in this art. In general, the incubation period will not exceed about an hour, and for most applications will lie in the range 30 minutes to one hour.

Localisation of the carrier material can be achieved in a wide variety of ways.

One simple embodiment is to allow particles to settle under gravity, although this necessitates a uniform suspension of the particles in the test medium being maintained, eg. by stirring, while the binding reaction is taking place. Centrifugation can be used as an alternative to gravitation. Alternatively, particles can be localised by passing a porous membrane or filter through the bulk of the test medium, thus sweeping the particles into a small volume. Another alternative is to use a particulate carrier material which, under the influence of magnetism or another externally-applied force, can be urged into a particular locality within the bulk of the test medium. The particles can be moved through a test medium by the ultrasonic generation of a drifting standing wave.

The solid phase can be made of any suitable carrier material. So-called "magnetic" (i.e. particles that can be induced to move through a liquid under the influence of a magnetic field) particles of a variety of suitable sizes, ranging for example from less than about 1 to more than about $300\mu$, are available commercially. For gravity separation, particle sizes of at least about $50\mu$, and not greater than about $300\mu$, are preferred, depending on the density of the material. Commercially-available oxirane acrylic beads are very suitable, especially for assays involving labelled haptens. Techniques for sensitising and/or coupling such solid phase carriers to binding reagents, such as immunoglobulins, are well known standard techniques in the art, and form no part of the present invention.

The range of analytes to which the invention can be applied is vast. By way of example only, the following can be mentioned: Steroid hormones, such as those involved during pregnancy or which are influential on fertility, e.g. progesterone and oestrogens. Urinary metabolites such as oestrone-3-glucuronide and pregnanediol-3-glucuronide. Peptide hormones such as human chorionic gonadotrophin, luteinising hormone and follicle stimulating hormone. Proteins such as urinary albumin, beta-2 microglobulins and retinol binding protein. High and low-density lipoprotein, the detection of which is valuable in cardiovascular monitoring, and cardiac troponin. Infectious disease markers, such as Rubella antibodies and Toxoplasmaantibodies. Immunoglobulins, such as monoclonal antibodies.

The test medium can thus be derived from many sources, especially body fluids such as urine, serum and plasma. The invention is especially applicable to the screening of the cell culture media for immunoglobulins, e.g. in the production of monoclonal antibodies.

The specific binding partners can be polyclonal or monoclonal antibodies or antigens, lectins, or hormones and their receptors. Such materials are well known in the art, many are available commercially, and their precise nature forms no part of the present invention.

If the sample suspected of containing the analyte is of variable nature, (e.g. serum and urine) or contains components which could interfere with the specific binding reactions or with the production of the observable signal, it can be advantageous to dilute the sample, e.g. with water or a buffer solution, before an assay method of the invention is applied, to reduce the likelihood of such interference.

An assay test kit in accordance with the invention can comprise, for example, a re-usable cell or plurality of such cells, complete with the signal sensing means (e.g. electrodes), or a disposable cell or cells incorporating a membrane through which the signal can pass to a re-usable sensing means, or a wholly disposable cell and signal sensing means combination. Appropriate reagents, e.g. the solid phase and other essential components, can also be supplied as necessary.

Various aspects of the invention are illustrated by the following Examples.

## Example 1

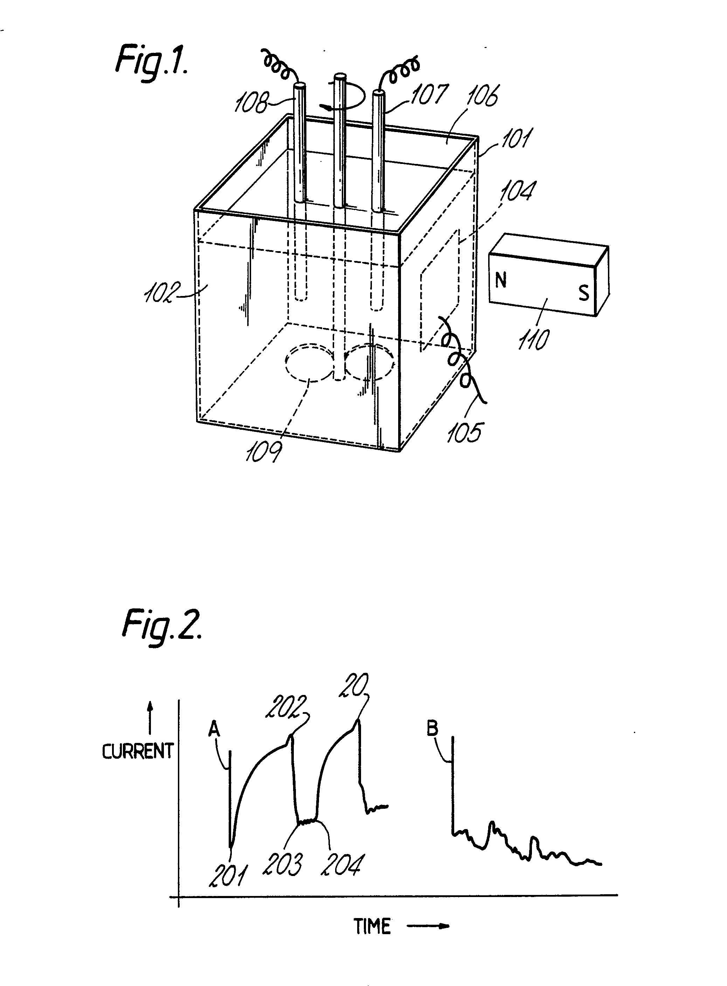

The principle of the invention can be demonstrated using the apparatus as depicted in Figure 1 of the accompanying drawings.

The apparatus comprises a cell 101 [depicted as being rectangular although in practice the shape will be immaterial] containing a volume 102 of liquid test medium. Mounted on the inner face of one wall 103 of cell 101 is a platinum foil electrode 104. Electrode 104 is linked via a connector 105 to a polarographic analyser [not shown] situated outside cell 101. Dipping into the text medium 102 from the open top 106 of cell 101 is a platinum counter electrode 107 and a silver/silver chloride reference electrode 108. A stirrer 109 is also provided to assist dispersion of material within the cell. A moveable bar magnet 110 can be placed at will outside the cell 100 immediately adjacent to the electrode 104.

The platinum foil electrode 104 can be set at voltage of plus 0.7 volts and is thereby capable of detecting the presence of hydrogen peroxide.

If the test medium contains magnetic microparticles bearing one member of an immunological binding pair, the binding reaction can be conducted while the particles are dispersed uniformly in the test medium. Thereafter, by introducing the magnet 110, the magnetic particles can be localised in the immediate vicinity of the detection electrode 104.

Using an apparatus as just described, the following experiment was conducted.

## MATERIALS AND METHODS

### Solid Phase

Magnetic particles, prepared as described by Pourfarzaneh et al (Methods of Biochemical Analysis, 1982 28, 267-295), were oxidised with periodic acid to generate surface aldehyde groups to which commercially available avidin was linked. This was achieved by treating 500mg of washed particles in 12.5ml distilled water with 400mg sodium periodate in 5ml of distilled water. The treatment was continued for one hour at room temperature in the dark, with end-over-end mixing. Excess periodate solution was removed and the oxidised particles then washed twice with distilled water and 3 times with borate buffer, pH 9.0, before the addition of 25ml of avidin solution (2mg/ml) in borate buffer (pH 9.0). This coupling reaction was maintained for one hour at room temperature on an end-over-end mixer, before unbound avidin was discarded and the particles washed five times in borate buffer. Residual aldehyde groups were eliminated first by reduction with an excess of sodium borohydride (which also stabilised the avidin-cellulose linkage), and then by reaction with 0.1m ethanolamine at pH 9.0. Finally, the particles were suspended in phosphate buffered solution containing 0.15% "Tween 20" (PBST) at a concentration of 500mg in 25ml. At each stage of the process, magnetic separation was used to remove reagent solutions and washing fluid from the particles. In subsequent use, this stock preparation was swirled vigorously immediately before samples were withdrawn to ensure that the particles were uniformly suspended.

Conjugate

Biotinylated glucose oxidase was obtained commercially as a freeze dried powder and contained approximately 20nmols biotin per mg protein. It was reconstituted as recommended and diluted in PBST immediately before use.

Catalase

Commercially available bovine liver catalase was obtained as a purified powder. Working solutions in PBS were prepared freshly each day.

Biotin

d-biotin was obtained commercially as a crystalline solid, and solutions of appropriate concentration were prepared by dilution from a reference solution consisting of an accurately weighed quantity of biotin dissolved in PBS.

Reaction Medium

The particles, conjugate and sample were suspended in PBS containing glucose at 1.0mg/ml and catalase at 2.5μg (approximately 25 units/ml). All of the solutions and reactants were free of sodium azide.

Assay Method

Solutions of biotin-glucose oxidase conjugate were prepared by diluting 100μl of reconstituted stock solution in 10ml of PBST alone (to give the maximum conjugate-binding signal) or 10ml PBST containing 100μg of free biotin (to give the absolute minimum, fully inhibited signal). To 100μl of these, 100μl of stock suspension of avidin-magnetic particles was added and, after a minimum of 10 minutes (to allow binding) the resultant mixtures were placed in the electrochemical cell with 3.8ml of reaction medium. At this point the polargraphic analyser and recorder were switched-on, to monitor the quantity of $H_2O_2$ reaching the electrode. Once the baseline had levelled out, the magnet was applied and the particles collected by magnetic attraction onto the surface of the electrode. The particles could be released into suspension again by removing the magnet and agitating the solution.

RESULTS

Line A in Figure 2 illustrates the typical trace obtained from the polarographic analyser. After the magnet had been introduced [point 201] the response rose rapidly by about 0.4μA, indicating a significant build-up of peroxide in the vicinity of the electrode. At point 202, the magnet was removed and the particles became dispersed quite rapidly back into the volume of the test medium. The response therefore fell to a background level 203 which was rising at a uniform slow rate associated with the gradual build-up of peroxide in the volume of the test medium which was not completely destroyed by the catalase. At point 204 the magnet was reintroduced, and an identical response obtained up to point 205, after which the magnet was again removed. The response thereafter returned to the background level, which had risen only slightly from the previous background. Although the net production of $H_2O_2$ within the cell as a whole remained the same, the asymmetric distribution of conjugate caused by binding to the solid phase and then localisation of solid phase at the point of detection, resulted in a distinct increase in the proportion of that $H_2O_2$ becoming available to the electrode.

A completely different trace (line B) was obtained when the test medium contained excess free unlabelled biotin. This competed successfully for the avidin and as a result, the magnetic particles were mostly carrying a complex which contained very little of the glucose oxidase-labelled biotin. In this instance the background level of peroxide detected by the electrode apparently fell during the course of the experiment. Localisation of the magnetic particles in the vicinity of the detection electrode did not result in any significant accumulation of peroxide, because the labelled biotin mostly remained dispersed in the volume of the test medium, and hence the distribution of the $H_2O_2$-generating glucose oxidase remained symmetrical, despite the location of the particles.

## Example 2

In this system all of the reagents, solutions and methods were the same as those with example 1, except for the form and arrangement of the electrode. A length of platinum wire was dipped into an acetone solution of cellulose acetate, and as it was removed, a layer of solution was left covering the entire area which had been submerged. The acetone was allowed to evaporate, leaving a continuous membrane of cellulose acetate covering the platinum, providing a barrier to potentially-interfering molecules but allowing $H_2O_2$ to pass through.

This coated wire was glued with polystyrene cement to a top corner of the cell. With this arrangement, the particles could be attracted on to the electrode by placing a pole of the magnet onto the outside of the corner. The particles then clustered around the electrode.

The experiment to demonstrate the effect of maximal and minimal binding was repeated in the same manner as in Example 1, with similar results being obtained except that magnitude of this response was higher, presumably because of a more efficient interaction between the electrode and the enzyme-carrying particles. This was in spite of the smaller surface area of the electrode and the presence of the intervening cellulose acetate membrane.

## Example 3

This example illustrates particle localisation by gravitation.

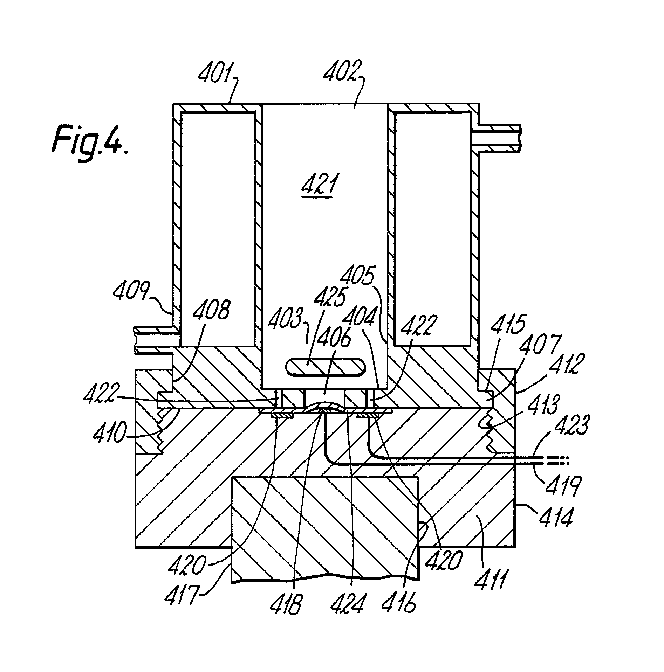

The apparatus used was a slightly modified Rank Oxygen Electrode and is depicted in cross-sectional elevation in Figure 4. This comprised an upright cylindrical water-jacketed sleeve 401 fully opened at the top end 402 and partially opened at the bottom end 403. The bottom end 403 is partially closed by an annular extension 404 of the inner face 405 of the sleeve, leaving a central circular orifice 406. The sleeve 401 has an external flange 407 running around the bottom end 408 of the outer wall 409 and the sleeve fits flushly on to the top surface 410 of a cylindrical base 411. The sleeve 401 and base 411 can be secured together by means of an outer ring 412, which is threaded internally to engage with a correspondingly threaded region 413 on the outside 414 of the base 411 and which has an internal annular lip 415 that fits over the flange 407 on the sleeve. The lower region of base 411 is centrally recessed 415 to accommodate a magnetic stirrer drive 417.

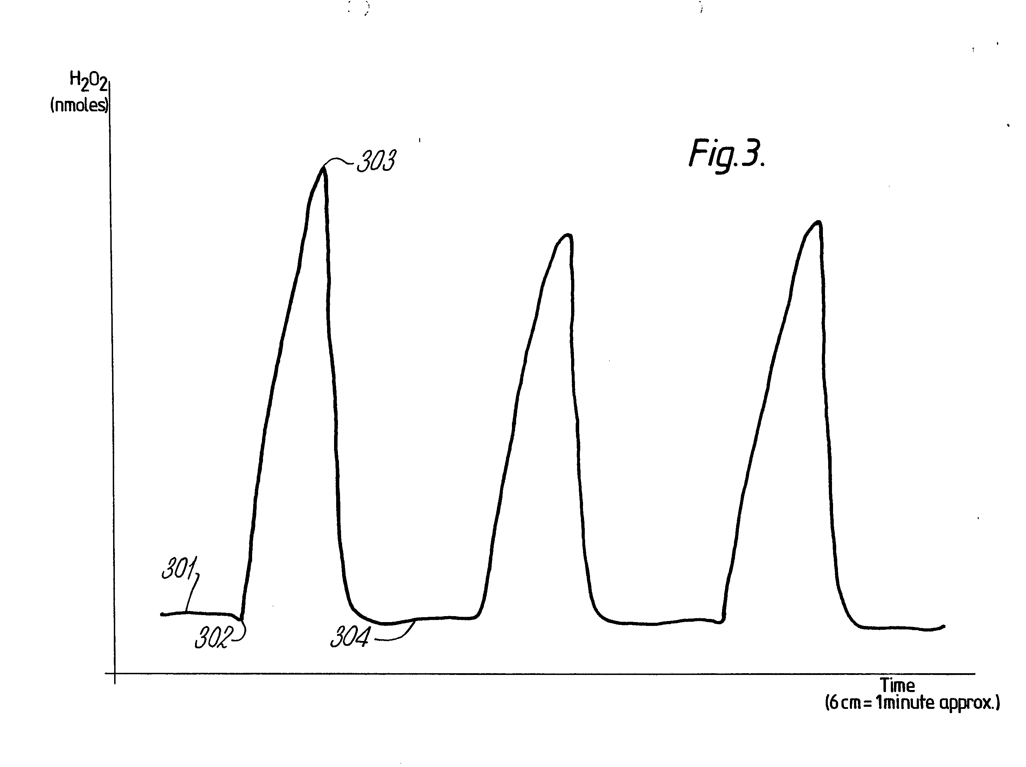

In the middle of the upper surface 410 of base 411, is a platinum button electrode 418 which in the assembled apparatus, lies in the central orifice 406 in the partially-closed bottom end 403 of sleeve 401. An electrical connector 419 passes from the electrode 418 through the base 411 to a polarographic analyser (not shown). A silver ring electrode 420 is mounted on base 411 concentrically with the platinum electrode 418 but spaced therefrom. In the assembled apparatus, the ring electrode 420 lies beneath the annular extension 404. In order for the ring electrode 420 to be readily contactable by any fluid contents in the cell 421 (formed by the sleeve 401 and base 411), a plurality of holes 422 were made in the annular extension 404 two of which are seen in Figure 3. A second electrical connector 423 passes from ring electrode 420 through base 411. A membrane 424 (made from standard laboratory dialysis membrane) is clamped between sleeve 401 and base 411 covering both electrodes.

The bar 425 of a magnetic stirrer lies in the cell 421 within the sleeve, and can be actuated by the stirrer head 417.

Materials and Methods

The solid phase was avidin from egg-white attached to commercially available epoxy-activated agarose (10 to 180 microns diameter) and containing 1-2 $\times$ 10$^6$/ml particles with the capacity of binding 20$\mu$g of biotin per ml.

The conjugate, catalase and biotin were exactly as used in Example 1.

The assay medium was prepared from the avidin-agarose (50$\mu$l of gel) in 1ml of phosphate buffered saline (PBS) containing the conjugate and biotin solution, and incubated at room temperature. For each experiment a 25$\mu$l aliquot of the pre-incubated gel/conjugate/biotin solution was added to 2.5ml of PBS containing 5mg/ml glucose and 10$\mu$g of catalase. This was equivalent to 10$\mu$l of gel and 1.25 of glucose oxidase in total. Stirring was continued while a base line H$_2$O$_2$ concentration was established, and the localised signal was measured after removing the magnetic stirrer bar with the aid of a hand-held magnet. The magnetic bar was replaced to stir the particles again to redistribute the components. Whenever the magnetic bar was removed, the particles sedimented very rapidly on to the membrane covering the electrodes.

A typical trace from the experiment is illustrated in Figure 3.

The "steady state" of hydrogen peroxide detected by the platinum electrode while the stirrer was in operation, is shown by the level base line 301. At point 302 the magnetic stirrer bar was removed from the cell and the particulate carrier material immediately settled under gravity. Hydrogen peroxide immediately began to accumulate in the immediate vicinity of the platinum electrode and the response rose rapidly. At point 303 the stirrer bar was reintroduced into the cell and the particulate material dispersed. The response thereafter fell rapidly to the original base line 304. The following cycles show that this effect was fully reproducable as the stirrer bar was successively removed and reintroduced.

The mean results of three determinations are given below in Table 1.

## TABLE 1

| Sample | Rate in nmoles/min of H$_2$O$_2$ |
|---|---|
| 10$\mu$g/ml biotin | 0 |
| 1$\mu$g/ml biotin | 23.7 |
| 100ng/ml biotin | 125.9 |
| 10ng/ml biotin | 145.3 |
| No biotin | 174.2 |

Example 4

This example demonstrates the determination of oestrone-3-glucuronide (E3G) as analyte.

The equipment used was the same as that described under Example 3 above, except that a mechanical top-driven stirrer was used in place of the magnetic stirrer; and the electrode system comprised a single flat disc platinum working electrode covering the entire bottom surface of the cell, plus a platinum counter electrode and a silver/silver chloride reference electrode both dipped into the cell from the top.

Solid Phase

Oxirane acrylic beads (epoxy-activated) were obtained commercially and were coupled to the IgG fraction of a rabbit anti-mouse serum. The antibody-linked beads were stored in phosphate buffered saline (PBS) pH 7.1.

## Conjugate

E3G was chemically attached to glucose oxidase by a modification of the SPDP method, in which the carboxyl group of the glucuronide was activated by reaction with a pyridyl thioamine derivative and a water soluble carbodiimide. The resultant conjugate was able to bind to monoclonal antibodies to E3G and had retained most of its glucose oxidase activity. It was stored as a solution in PBS at +4°C, and aliquots were diluted to working strength as necessary each day.

## Monoclonal Antibodies

Mouse monoclonal antibodies with specificity for free E3G were prepared by conventional means with E3G/BSA conjugates as the immunogen. The hybridoma cells were grown in serum-free culture medium and the monoclonal antibodies were isolated by ion-exchange chromatography.

## Calatase

Commercially available bovine liver catalase was obtained as a purified powder. Working solutions in PBS were prepared freshly each day.

## Assay Method

To 100μl of an E3G-containing test sample, was added 50μl of PBS containing the E3G/glucose oxidase conjugate (one 50th dilution) plus 650μg per ml of the anti-E3G antibody. After a 30 minute incubation at room temperature, 20μl of the solid phase suspension (1mg) was added to the reaction mixture and a whole mixture was subjected to end-over-end mixing for a further 30 minutes. At this point, glucose and catalase were added at working strength (150mM and 6,000 units respectively) in 1.2ml of PBS and the whole sample was transferred to the cell.

The contents of the cell were stirred while a steady-state baseline peroxide level was established. Stirring was stopped and the particulate solid phase allowed to settle onto the membrane covering the electrode. The resulting signal was recorded as a rate of peroxide production, in arbitrary units, derived from the trace of a strip-chart recorder. To establish a standard curve a series of samples was prepared with a range of concentrations of E3G (from 3ng per ml to 1,000ng per ml) and the assay procedure carried out with each in triplicate.

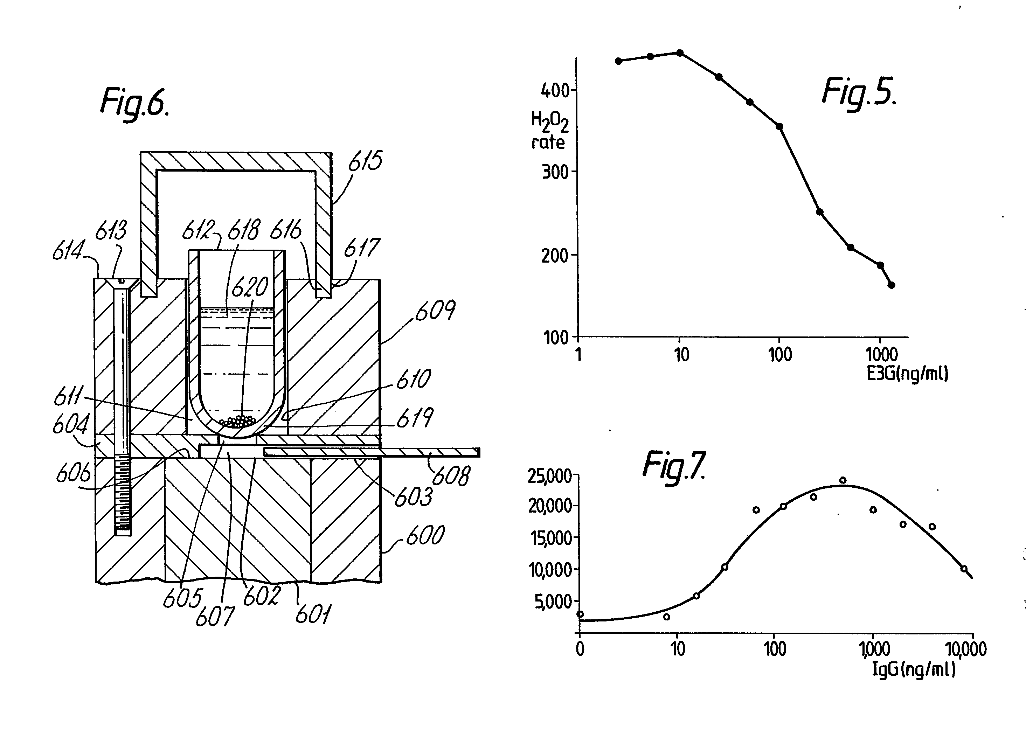

Figure 5 illustrates the results obtained.

## Example 5

This example illustrates the generation of a detectable hydrogen peroxide signal by localisation of a particulate carrier onto a gel layer in which the peroxide can be detected by means of a chemical reaction resulting in the emission of visible light from the gel. While the carrier material remains dispersed in the sample, free catalase enzyme is used to destroy the peroxide and eliminate any signal from the bulk sample.

A set of small closable transparent plastics sample tubes are used as the basic assay cells. The exact size is not important, but usually these are about 1cm in diameter and about 3cm in height. A small quantity (e.g. 0.15ml) of agarose detector gel is cast into the base of each tube.

## Materials

### Solid phase

Commercially-available epoxy-activated oxirane acrylic beads (size range 100-200μm), sensitised with avidin from hen's eggs, at a level of 16mg avidin per 10g dry weight of beads. The avidin-linked beads can be stored in phosphate-buffered saline solution (PBS) at pH 7.1.

## Conjugate

Biotinylated glucose oxidase obtained commercially as a freeze dried powder and containing approximately 20nmols biotin per mg protein. It is reconstituted as recommended and diluted in PBST immediately before use.

## Biotin

d-biotin obtained commercially as a crystalline solid, and solutions of appropriate concentration prepared by dilution from a reference solution consisting of an accurately weighed quantity of biotin dissolved in PBS. The biotin concentration is varied, e.g. from 5ng/ml to 100 ng/ml.

## Detector Gel

A quantity of gel, based on 1% (weight by volume) agarose in Tris buffer at pH 8.5, is prepared, containing 1.25mM luminol and 0.005 units of encapsulated horseradish peroxide enzyme. The encapsulated HRP can be obtained commercially and consists of enzyme entrapped in polyamide micro capsules (20-80μ diameter) at a level of 0.656 units per mg of wet solids. The capsule membranes are semipermeable, allowing free diffusion of small molecules such as hydrogen peroxide but retaining molecules of molecular weight greater than 10,000.

## Assay Method

A sample of the avidin-sensitised beads is centrifuged and the supernatant removed. A 0.05% solution of keltrol in buffer solution is added to produce a 50/50 suspension of beads. A 5μg per ml solution of glucose oxidase biotin conjugate from the stock reagent is prepared in 0.05% keltrol in buffer. To each of a number of microfuge tubes is added 100μl of the bead suspension, 500μl of the conjugate and 500μl of biotin solution. Each tube is capped and placed on an end-over-end mixer for 2 hours at ambient temperature.

After incubation 50μl of beads in 2ml of PBS buffer containing 1mg/ml glucose and commercially-available bovine liver catalase enzyme at a level of about 25 units/ml, is added to each tube. The beads settle onto the gel surface, and in the case of samples which contain little or no free biotin, a readily visible blue light is emitted by the gel layer.

## Example 6

This example illustrates the generation of a chemiluminescent signal and the use of the localisation of a particulate carrier material in the assaying of a monoclonal antibody. This procedure can be used in the screening of cell culture media, e.g. for the presence of immunoglobulins such as monoclonal antibodies.

## Materials

## Solid Phase

Commerically-available macroporous polystyrene particles (Dyno: Dynospheres XP-6006), average size 2.6μm, within which is an even distribution of iron oxide. These uniform, spherical beads have a hydroxyethylmethacrylate based outer shell derivitised with pendant tosyl groups. The tosyl moieties can readily undergo nucleophilic substitution by amino groups such as are present on the outer surface of a protein, leading to immobilisation. These were sensitised with a commercially-available rabbit anti-mouse monoclonal antibody (Z259 obtained from Dako), by the following procedure.

To an aliquot of a suspension containing Dynospheres XP-6006 (10mgs) was added boric acid buffer (0.005M, pH 9.5, 0.5ml). To this was then added sheep IgG solution (200μg in 1ml PBS, pH 7.2) followed by boric acid buffer (0.5M, adjusted to pH 9.5 with NaOH, 0.5ml).

This was left overnight on an orbital shaker at room temperature to couple. The beads were then sedimented with a magnet and the protein solution aspirated. They were washed once with PBS (5ml), and the coupling procedure is repeated, but this time using BSA (20mgs) to couple to any unreacted tosyl groups. The beads were then washed overnight in phosphate buffer (0.25M $KH_2PO_4$, adjusted to pH 8 with NaOH containing 0.5% BSA and 0.1% Tween 20, 5ml) to remove any adsorbed IgG from the bead surface. Finally, the beads were washed in the same buffer (2 $\times$ 5ml) and then washed in PBS (2 $\times$ 5ml) and stored in a known volume of PBS containing 0.1% BSA + 0.01% Thimerosal preservative at 4°C.

## Conjugate

Commercially available sheep anti-mouse IgG monoclonal antibody conjugated with horseradish peroxidase, A6782 obtained from Sigma.

## Analyte

An anti-alpha HCG mouse monoclonal immunoglobulin in a range of standard concentrations from 0 to 8μg per ml in whole culture medium (10% horse serum/HAZ).

## Method

All reagents, other than the analyte sample, were in 0.1M Tris/HCl pH 8.6 buffer. 20μl of 0.625% (by volume) suspension of the rabbit anti-mouse sensitised magnetic particles were dispensed into a tube along with 100μl of an analyte solution and 100μl of the conjugate at 1/200 dilution. This mixture was incubated at room temperature on a standard laboratory shaker for 30 minutes.

After this time, the following reagents was added to the tube in the following order of addition:

250μl of 10mM luminol
250μl of 5mM para-iodophenol
15μl of commercially-available aqueous black drawing ink (selected not to interfere with the chemiluminescent reaction)
250μl of 100mM hydrogen peroxide

The contents of the tube were thoroughly mixed and the magnetic solid phase was then localised at the base of the tube by placing a permanent magnet adjacent the underside of the tube for five minutes. The tube was then removed from the magnet and placed carefully (so as not to dislodge the pellet of localised particles) into a light sensor.

The essential features of the optical sensing device used in this example are illustrated diagrammatically in Figure 6, which represents a cross-sectional elevation.

The device comprises an upright open-topped cylindrical housing 600 accommodating within it a photomultiplier tube 601. The window 602 of photomultiplier tube 601 lies flush with the top 603 of housing 600 and is directed vertically upwards. The constructional details of the photomultiplier tube 601 are not shown, as these are irrelevant to the invention.

Superimposed on the top 603 of housing 600 is a flat circular plate 604 of the same diameter as housing 600. Plate 604 is pierced centrally by a small circular aperture 605 directly above window 602 of photomultiplier tube 601. The lower surface 606 of plate 604 incorporates a horizontal slot which leaves a narrow gap 607 between plate 604 and the top 603 of cylindrical housing 600, into which gap a horizontally slidable shutter 608 can be moved. Shutter 608 communicates with the outside of the device and hence can be opened and closed manually. Shutter 608 is depicted in the open position. When fully inserted, shutter 608 blocks aperture 605 and effectively prevents any light reaching window 602 of photomultiplier tube 601.

Superimposed on plate 604 is an annular body 609 of the same external diameter as housing 600 and plate 604, and having its vertical cylindrical axis on the same line as that of aperture 605 and photomultiplier tube 601. The inner surface 610 of annular body 609 is of greater diameter than aperture 605, and provides a recess 611 into which an assay tube 612 can be fitted (see below).

Annular body 609 and circular plate 604 are secured in place on top of cylindrical housing 600 by means of a plurality of bolts 613 (only one of which is shown in the drawing), which extend downwards from the top 614 of annular body 609 and into cylindrical housing 600.

A readily removable light-tight cover 615 is provided on the top 614 of annular body 609 cylindrical housing, the bottom rim 616 of which engages in an annular groove 617 in top 614, allowing cover 615 to prevent light entering recess 611.

As seen in Figure 6, recess 611 within annular body 609 is occupied by a round-bottomed glass tube 612 in which a liquid sample 618 has been placed for analysis. The bottom 619 of tube 612 rests in aperture 605 in disc 604. A particulate solid phase carrier material 620 used in the experiment is shown localised at the bottom 619 of glass tube 612. Chemiluminescent light produced in the vicinity of the particulate carrier material can pass through the glass wall at the bottom of the tube and through aperture 605 in disc 604 and directly to

In operation, after all of the analytical reagents have been mixed together in the tube 612 and the magnetic particles localised at the bottom, the tube is placed inside recess 611 and cover 615 placed on body 609. Shutter 608 is opened for a predetermined time, e.g. 10 seconds, and the reading from the photomultiplier tube is noted.

The results, expressed in orbitrary units, of a series of experiments using different known analyte concentrations are given in Table 1 below and are also depicted graphically in Figure 7 of the accompanying drawings. The falling results at high analyte concentrations is due to the "hook" effect.

## Table 1

| Analyte concentration | Optical reading |
|---|---|
| 0 (Buffer) | 2934 |
| 7.8 | 2641 |
| 15.6 | 6006 |
| 31.25 | 10608 |
| 62.5 | 19562 |
| 125 | 20302 |
| 250 | 21804 |
| 500 | 24330 |
| 1000 | 19806 |
| 2000 | 17393 |
| 4000 | 17220 |
| 8000 | 10310 |

## Claims

1. An assay method for determining qualitatively and/or quantitatively the presence of an analyte in a sample by means of specific binding, wherein the sample is contacted with a moveable solid phase carrier material, such as a particulate carrier, on which is immobilised a first binding reagent having specificity for the analyte and with a labelled specific binding reagent which can participate in either a "sandwich" or a "competition" reaction with the first reagent in the presence of the analyte, and in which method, following an incubation period sufficient to allow the reaction to take place, the moveable solid phase carrier material is moved within the assay medium to a location adjacent a signal sensing means, the label generating a signal continuously in the assay medium and the magnitude of the signal generated in the vicinity of the sensing means being used as a measure of the extent to which the binding reaction has occurred.

2. An assay method according to claim 1, which utilises a specific binding reagent which is labelled such that the label continuously generates a signal in the assay medium, which signal is suppressed while the labelled binding reagent is uniformly dispersed in the assay medium, and in which method, following the binding reaction, the particulate carrier material is localised in an environment having insufficient signal-suppressing, ability to overcome the locally-generated signal, and hence a detectable signal can be generated the nature and/or magnitude of which is dependent on the extent of binding that has taken place between the analyte and the specific binding reagent immobilised on the particles.

3. An assay method according to claim 1 or claim 2, wherein the signal detected by the sensing means is visible light originating from a chemiluminescent reaction.

4. An assay method according to claim 3, wherein the label generates hydrogen peroxide in the assay medium and the peroxide is detected via its participation in a chemiluminescent reaction.

5. An assay method according to claim 4, wherein the label is a peroxide-destroying enzyme which engages in a chemiluminescent reaction.

6. An assay method according to claim 1 or claim 2, wherein the label generates hydrogen peroxide in the assay medium and the peroxide is measured electrochemically.

7. An assay method according to any one of the preceding claims, wherein the assay medium incorporates a masking or quenching agent which suppresses signal generated in regions of the assay medium remote from the signal sensing means.

8. An assay method according to claim 7, wherein the detectable signal is chemiluminescent light and the masking or quenching reagent renders the assay medium opaque or at least insufficiently translucent for light from regions remote from the signal sensing means to reach the signal sensing means.

9. An assay method according to claim 7, wherein the masking or quenching reagent is a chemical reagent which consumes an essential component of the signal generating system.

10. An assay method according to claim 9, wherein the label produces hydrogen peroxide in the assay medium and the consumer reagent is catalase enzyme.

11. An assay method according to claim 5, wherein the label is horseradish peroxidase and the assay medium incorporates a peroxide, such as hydrogen peroxide, and a chemiluminescent 2,3-dihydro-1,4-phthalazinedione, such as luminol, and the assay medium optionally also incorporates an enhancer for the chemiluminescent reaction, such as para-iodophenol, para-phenylphenol and/or 2-chloro-4-phenylphenol.

12. An assay method according to claim 1, wherein the assay medium incorporates:

a) a first population of localisable particles on which is immobilised a binding reagent having specificity for the analyte;

b) a second specific binding reagent labelled such that it continuously produces within the assay medium a precursor for a chemiluminescent reaction;

c) a consumer reagent dispersed in the test medium which competes effectively for the precursor and thereby inhibits the production of chemiluminescence while the labelled specific binding reagent remains dispersed in the assay medium; and

d) a second population of localisable particles bearing or containing a reagent which can react with the precursor to generate chemiluminescent light; in which assay method co-localisation of the two particle populations adjacent a light detection means enables observation of chemiluminescence produced in an amount dependant on the extent to which the specific binding reagent immobilised on the first population of particles has become bound to the analyte.

13. An assay method according to claim 12, wherein the label is glucose oxidase, the assay medium contains glucose and a chemiluminescent 2,3-dihydro-1,4-phthalazinedione such as luminol, the consumer reagent is catalase enzyme, the reagent associated with the second population of localisable particles is horseradish peroxidase, and the assay medium optionally also an enhancer such as incorporates para-iodophenol.

14. An assay method according to claim 1, wherein a chemical signal is detected by localising a particulate carrier adjacent a surface containing or carrying a reagent which interacts with the chemical signal.

15. An assay method according to claim 14, wherein the chemical signal is hydrogen peroxide and the surface is a gel body containing horseradish peroxidase and a chemiluminescent 2,3-dihydro-1,4-phthalazinedione luminol, and optionally also an enhancer such as para-iodophenol.

16. An assay method according to any one of the preceding claims, applied to the screening of cell culture media.

# Fig.1.

# Fig.2.

Fig.3.

H₂O₂
(nmoles)

301
302
303
304

Time
(6 cm = 1 minute approx.)

0 247 796

Fig.4.

Fig.6.

Fig.5.

Fig.7.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,Y | WO-A-8 604 684 (LABSYSTEMS OY) * Whole document * | 1-16 | G 01 N 33/546 |
| | --- | | G 01 N 33/543// |
| Y | WO-A-8 601 899 (UNILEVER PLC) * Whole document * | 1-16 | G 01 N 33/553 G 01 N 27/46 G 01 N 33/535 G 01 N 33/533 |
| Y | EP-A-0 122 059 (SYVA CO.) * Abstract; page 4, line 9 - page 6, line 22; claims 1-14 * | 1-16 | |
| Y | EP-A-0 075 379 (SYVA CO.) * Whole document * | 1-16 | |
| A | US-A-4 115 535 (I. GIAEVER) * Whole document * | 1 | |
| | | | TECHNICAL FIELDS SEARCHED (Int. Cl.4) |
| A | EP-A-0 170 446 (SERONO DIAGNOSTICS LTD) | | G 01 N |
| A | EP-A-0 169 434 (LABSYSTEMS OY) * Whole document * | 1-16 | |
| A | EP-A-0 070 686 (STANDARD OIL CO.) | | |
| D,Y | EP-A-0 149 565 (AMERSHAM INTERNATIONAL PLC) * Whole document * | 1-16 | |
| | --- -/- | | |

The present search report has been drawn up for all claims

| Place of search | Date of completion of the search | Examiner |
|---|---|---|
| THE HAGUE | 17-08-1987 | OSBORNE H.H. |

| | DOCUMENTS CONSIDERED TO BE RELEVANT | | Page 2 |

| Category | Citation of document with indication, where appropriate, of relevant passages | Relevant to claim | CLASSIFICATION OF THE APPLICATION (Int. Cl.4) |
|---|---|---|---|
| Y | WO-A-7 900 882 (SYVA CO.) * Whole document * | 1-16 | |
| Y | EP-A-0 165 072 (MAST IMMUNOSYSTEMS INC.) * Abstract; pages 24,25 * | 1-16 | |

TECHNICAL FIELDS
SEARCHED (Int. Cl.4)

The present search report has been drawn up for all claims

| Place of search | Date of completion of the search | Examiner |
|---|---|---|
| THE HAGUE | 17-08-1987 | OSBORNE H.H. |